Europäisches Patentamt

European Patent Office

Office européen des brevets

(11) Publication number: **0 410 348 A1**

# EUROPEAN PATENT APPLICATION

(21) Application number: 90114076.4

(22) Date of filing: 23.07.90

(51) Int. Cl.⁵: **A61K 31/585**, A61K 7/48

(30) Priority: 26.07.89 US 385751

(43) Date of publication of application:
30.01.91 Bulletin 91/05

(84) Designated Contracting States:
AT BE CH DE DK ES FR GB GR IT LI LU NL SE

(71) Applicant: G.D. Searle & Co.Co.
P.O. Box 5110
Chicago Illinois 60680(US)

(72) Inventor: Kim, Chung Eun
239 Valley View Drive
Wilmette, Illinois 60091(US)
Inventor: Morales, Olga Iris
1330 Paddock Drive
Wheeling, Illinois 60090(US)
Inventor: Pazdur, James John
29451 Hawthorn Lane
Libertyville, Illinois 60048(US)

(74) Representative: Beil, Walter, Dr. et al
BEIL, WOLFF & BEIL Rechtsanwälte
Adelonstrasse 58 58
D-6230 Frankfurt am Main 80(DE)

(54) Topical spironolactone composition.

(57) The invention relates to a topical spironolactone composition for the treatment of acne, hirsutism and other conditions caused by excess androgenic activity.

# TOPICAL SPIRONOLACTONE COMPOSITION

## FIELD OF THE INVENTION

The present invention relates to a topical spironolactone composition useful for treating acne, hirsutism, and other disorders caused by excess androgenic activity. The topical composition permits the administration of spironolactone at the affected skin site in order to reduce the likelihood of subjecting the patient to the systemic effects of spironolactone. The composition of the present invention comprises a topical formulation of spironolactone which has the advantages of being very stable during long-term storage and having good cosmetic acceptability. Spironolactone is practically insoluble in water, and its poor solubility characteristics make it difficult to develop a cream formulation which has good stability, good cosmetic acceptability, and good anti-androgenic activity.

## BACKGROUND OF THE INVENTION

U.S. Patent No. RE 32112 (Shapiro) discloses a composition for effectively suppressing excess androgenic activity which occurs at a skin site, which consists essentially of spironolactone in an amount of from about 0.25 weight percent to about 5.0 weight percent in a suitable carrier. Examples of suitable carriers are alcohol, urea, mineral oil or white petrolatum. A solubilizer such as stearyl alcohol or cetyl alcohol in an amount of about 1.5 weight percent to 3.0 wt.% can be used to solubilize the spironolactone and a viscosifier such as 3.0 to 6.0 weight percent cottonseed oil can also be used in the composition as can a preservative such as 0.25 to 0.5 weight percent methyl hydroxy benzoate. The composition is useful for treating acne, hirsutism and baldness caused by excess androgenic activity. The stearyl alcohol or cetyl alcohol disclosed as solubulizers in this patent have the disadvantage of not being good solvents for spironolactone. Furthermore, Shapiro lists alcohol as a carrier and many low carbon alcohols such as ethanol and isopropyl alcohol are undesirable because they tend to cause degradation of spironolactone.

U. S. Patent No. 4,543,351 (Messina) discloses a spironolactone-containing composition for combating acne in the form of a cream to be applied to human skin. The amount of spironolactone in the cream may be from 0.1 to 10 weight percent. The carrier in which the spironolactone is incorporated is a mixture of (1) from 10 to 25 weight percent of a mixed polyethylene glycol and polyoxyethylene glycol ester of a higher saturated fatty acid, (2) from 2 to 10 weight percent of a saturated polyoxyethylene glycolglyceride, and (3) from 60 to 75 weight percent of water. Liquid paraffin may be added to give the desired consistency. This composition also has certain disadvantages. For example, it does not contain any ingredient which would act as a solvent for spironolactone. The ingredient Tefose 63 (polyethylene glycol and polyoxyethylene glycol palmito stearate) when combined alone with spironolactone tends to degrade the spironolactone and would therefore be expected to be less satisfactory as an ingredient for a spironolactone-containing composition.

European patent application EP 0 028 525 discloses a topical hair treatment agent to promote the growth of hair and prevent dandruff that is characterized in that it contains spironolactone as the active component. The hair treatment agent is used in solution form as a liniment. It contains 0.01 to 2 weight percent spironolactone along with solvents which may be monohydric aliphatic alcohols such as ethanol and propanol or di- and polyhydric alcohols such as glycerol, propylene gylycol and polyethylene glycol. Spironolactone is unstable upon long term storage with these ingredients; and, therefore, this formulation is undesirable. When spironolactone breaks down an unpleasant mercaptan odor results. Thus ingredients which tend to degrade spironolactone are undesirable.

U.S. Patent No. 3, 711,602 discloses a topical ophthalmic formulation containing about 0.1 to 0.75 weight percent spironolactone along with polyethylene glycol, dimethyl sulfoxide (DMSO) and water. The DMSO is said to enhance penetration of the active ingredient.

FR2588755-A1 discloses a topical composition for the treatment of alopecia androgenetica comprising 1 to 10% spironolactone by weight in an alcohol solution preferably 1/3 alcohol 90° and 2/3 isopropyl alcohol An example of a formulation of the invention has the following ingredients:

EP 0 410 348 A1

| Spironolactone | 1 to 10% by weight |
|---|---|
| Alcohol 90° | about 20% by weight |
| Isopropyl alcohol | about 60% by weight |
| DMSO (dimethyl sulfoxide) | about 2.5% by weight |
| Distilled water q.s.p. | 100% by weight |

This formulation is undesirable because the alcohols used in this formulation would degrade spironolactone to canrenone, and the thio group which comes off spironolactone when it is degraded forms a mercaptan which has a disagreeable odor.

F.O. Rey, et. al. J. ENDOCRINOL. INVEST, 11 :273 (1988) discusses the lack of endocrine systemic side effects after topical application of spironolactone in man using a cream composed of purified water 66.35%, propylene glycol 12%, vaseline oil 8%, spironolactone 5%, cetostearyl alcohol 4.5%, cetomacronol 1000 (i.e. the polyethylene glycol ether of cetyl alcohol that conforms to the formula $CH_3(CH_2)_{14}CH_2-(OCH_2CH_2)_nOH$ where n has an average value of 20) 4%, adjuvants 0.65%. Since spironolactone tends to be unstable upon long term storage with propylene glycol and polyethylene glycol ether of cetyl alcohol, this would not be a desirable formulation.

## BRIEF SUMMARY OF THE INVENTION

The present invention relates to a topical spironolactone composition which is useful for treating acne, hirsutism, and other disorders caused by excess androgenic activity such as alopecia and seborrhea. The spironolactone-containing topical formulation is applied directly to the skin in order to treat the disease. The topical formulation is effective at the skin site, but has the advantage of reducing or avoiding the systemic side effects of spironolactone which would occur with oral or parenteral administration. The spironolactone composition of the present invention also has the advantages of being particularly stable upon long-term storage and having good cosmetic appearance and acceptability The composition contains from about 1 to about 12 weight percent of spironolactone as the active ingredient along with a suitable solvent for spironolactone such as diisopropyl adipate, emulsifying agents. such as cetearyl alcohol, sodium lauryl sulfate, glycerol monostearate, and polyoxyethylene stearate, buffering agents, preservatives such as methyl paraben and propyl paraben, an ointment base such as petrolatum, a noncomedogenic liquid carrier such as mineral oil, and water.

## DETAILED DESCRIPTION OF THE INVENTION

The topical spironolactone formulation of the present invention comprises an effective amount of spironolactone in a mixture of (1) emulsifying agents, (2) a suitable solvent for spironolactone, (3) buffering agents sufficient to maintain a pH of from about 4.0 to about 6.0 and (4) water. In addition an ointment base and a noncomedogenic liquid carrier may be employed to give the desired consistency. An effective amount of preservative may also be added.

A preferred composition comprises about 1.0 to 12.0 wt. percent of spironolactone along with (1) about 2.5 to 18.5 wt. percent of a mixed of cetearyl alcohol and sodium lauryl sulfate and a mixed glycerol monostearate and polyoxyethylene stearate, (2) about 0.5 to 6.0 wt. percent diisopropyl adipate or $C_8$-$C_{10}$ ethoxylated glyceride (3) about 0.23 to 2.3 wt. percent buffering agents; and (4) water. To the above may be added a noncomedogenic liquid carrier such as mineral oil and an ointment base such as petrolatum to produce an emulsion having the desired consistency. A preservative such as methyl and/or propyl paraben or other suitable preservative may be added.

In one embodiment the topical spironolactone composition of the present invention comprises:

about 1.0 to 12.0 wt. percent spironolactone;

about 2.5 to 18.5 wt. percent emulsifying agents;

about 0.5 to 6.0 wt. percent solvent;

about 50 to 80 wt. percent water; and sufficient buffering agents to maintain a pH of from about 4.0 to about 6.0.

3

In addition, to produce the desired consistency, the following ingredients can be included in the above formulation:

about 1.0 to 12.0 wt. percent of a non-comedogenic liquid carrier;

about 1.0 to 15.0 wt. percent of an ointment base.

From about 0.04 to about 0.6 wt. percent of preservative may also be added.

A preferred formulation of the present invention comprises:

1.0 to 12.0 wt. percent spironolactone;

1.5 to 12.0 wt. percent of a mixture of cetearyl alcohol and sodium lauryl sulfate;

1.0 to 6.5 wt. percent of a mixture of glycerol monostearate and polyoxyethylene stearate;

1.0 to 12.0 wt. percent mineral oil;

0.5 to 6.0 wt. percent diisopropyl adipate;

1.0 to 15.0 wt. percent petrolatum;

0.02 to 0.3 wt. percent propyl-p-hydroxybenzoate;

0.02 to 0.3 wt. percent methyl-p-hydroxybenzoate;

0.13 to 1.3 wt. percent of a weak acid salt;

0.1 to 1.0 wt. percent of a weak acid;

50 to 80 wt. percent water

A more preferred formulation of the present invention comprises:

1.0 to 12.0 wt. percent spironolactone;

7.2 to 10.8 wt. percent of a 90:10 mixture of cetearyl alcohol and sodium lauryl sulfate;

4.0 to 6.0 wt. percent of a 50:50 mixture of glycerol monostearate and polyoxyethylene stearate;

4.8 to 7.2 wt. percent light mineral oil;

3.6 to 5.4 wt. percent diisopropyl adipate;

8.4 to 12.6 wt. percent white petrolatum;

0.04 to 0.06 wt. percent propyl-p-hydroxybenzoate;

0.12 to 0.18 wt. percent methyl-p-hydroxybenzoate;

0.53 to 0.78 wt. percent sodium citrate dihydrate;

0.4 to 0.6 wt. percent citric acid monohydrate;

55 to 70 wt. percent purified water USP

The present invention relates to a topical spironolactone composition for use in the treatment of acne and other disorders characterized by excess androgenic activity such as hirsutism seborrhea and some types of baldness. The topical spironolactone composition is to be applied to the skin in the affected area. The use of the topical spironolactone composition of the present invention has the advantage of enabling the acne or other condition to be treated at the skin site where the excess androgenic activity occurs thus reducing the likelihood of subjecting the patient to the systemic effects of spironolactone which would occur with oral or parenteral administration. The formulation has the ability to penetrate the skin and stay there with very little systemic absorption of the spironolactone active ingredient, which is a desirable characteristic. Treatment with the formulation of the present invention is usually accomplished by applying it to completely cover the affected area. The usual frequency of application is twice daily, although this may vary depending on the particular patient and the severity of the condition being treated. The composition is for topical administration to warm-blooded animals, including man, and contains as the active anti-androgenic ingredient an effective amount of spironolactone.

An additional advantage of the topical spironolactone composition of the present invention is its stability upon long-term storage. Spironolactone is insoluble and typically unstable in aqueous systems. Many alcohols, particularly alcohols having a low number of carbon atoms, degrade spironolactone. In the formulation of the present invention, spironolactone is very stable and would be expected to remain stable for at least two years. The use of a solvent such as diisopropyl adipate which is a good solvent for spironolactone but does not readily degrade it and the maintenance of the pH at from about 4 to about 6 using an appropriate buffering system as well as the use of emulsifiers which will work in an acid pH all contribute to the stability of the spironolactone composition.

Examples of some weak acids which can be utilized in buffering the formulation of the present invention include, but are not limited to, citric acid, citric acid monohydrate, boric acid, and phosporic acid.

Examples of some acid salts which can be used in buffering the formulation include, but are not limited to, sodium citrate, sodium citrate dihydrate, monopotassium phosphate, and disodium phosphate. One skilled in the art would be aware that other weak acids and acid salts could also be used to maintain adequate buffering and, therefore, the above listings are not intended to be limiting.

Preservatives which are suitable for use in the formula include, but are not limited to, p-hydroxybenzoates such as methyl p-hydroxybenzoate, ethyl p-hydroxybenzoate, propyl p-hydroxybenzoate and butyl p-hydroxybenzoate as well as benzoic acid and imidizolidinyl urea.

A suitable solvent is one which readily dissolves spironolactone but does not readily degrade it to canrenone. Many alcohols, especially low carbon chain alcohols are undesireable as solvents because of their tendency to degrade spironolactone to canrenone and release foul smelling mercaptans. A solvent other than diisopropyl adipate which would be suitable for use in the present composition is $C_8$-$C_{10}$ ethoxylated glyceride which is available as Labrafac hydrophil WL1219 from Gattefosse, Sait Priest Cedex, France.

A preferred acid is citric acid monohydrate, A preferred acid salt is sodium citrate dihydrate.

The cosmetic acceptance of the formulation may be enhanced by using a "low odor" type of spironolactone such as spironolactone, USP, micronized which has been purified to reduce odors associated with mercaptans. A preferred water for use in the composition is purified water USP.

The amount of spironolactone in the composition may vary from about 1 weight percent to about 12 weight percent. In the preferred formulations the amount of spironolactone may vary from about 1.0 weight percent to about 5.0 weight percent with about 5.0 weight percent being most preferred.

The carrier in which the spironolactone is contained is an oil in water buffered emulsion comprising a mixture of cetearyl alcohol and sodium lauryl sulfate, preferably as a mixture of about 90% cetearyl alcohol and about 10% sodium lauryl sulfate such as Lanette SX, which is available from Henkel Corp., 300 Brookside Ave., Ambler, PA 19002; glycerol monostearate and polyoxyethylene stearate, preferably as a mixture of about 50% glycerol monostearate and about 50% polyoxyethylene stearate such as Arlacel® 165 which is available from ICI Americas, Inc., Cherry Lane, P.0. Box 231, New Castle, Delaware 19720; diisopropyl adipate which serves as a solvent for spironolactone; appropriate buffers such as sodium citrate dihydrate and citric acid monohydrate; and water. In addition petrolatum and mineral oil may be added to give the desired consistency and preservatives such as propyl-p-hydroxybenzoate and/or methyl-p-hydroxybenzoate may also be added.

A preferred petrolatum is white petrolatum although other petrolatums can also be used. A preferred mineral oil is light mineral oil although other mineral oils can also be used.

Cetearyl alcohol is a mixture of fatty alcohols consisting predominantly of cetyl and stearyl alcohols (See CTFA Cosmetic Ingredient Dictionary , 3rd Ed. 1982). A preferred polyoxyethylene stearate is PEG-100 stearate which is the polyethylene glycol ester of stearic acid that conforms to the formula;

$$CH_3(CH_2)_{16}\overset{\text{O}}{\overset{\|}{C}}-(OCH_2CH_2)_nOH$$

Where n has an average value of 100.

The composition of the present invention is manufactured in a conventional manner by thoroughly mixing the ingredients, preferably at elevated temperatures. Preferably the spironolactone is added to the oil phase. The ingredients are thoroughly mixed so that the product is homogeneous. Processing equipment suitable for preparing the composition is known in the art.

The topical composition of the present invention is prepared by first heating the oil phase ingredients until melted then adding spironolactone to the oil phase and homogenizing. The aqueous phase ingredients are heated in a separate container, then the oil phase and aqueous phase are combined and emulsified under appropriate temperature and pressure conditions. The emulsified composition is then cooled and blended and transferred to appropriate containers.

For example, a topical spironolactone composition of the present invention may be prepared by heating a mixture of the following oil phase ingredients until melted: cetearyl alcohol/sodium lauryl sulfate blend (for example, LANETTE SX), glycerol monostearate/ polyoxyethylene stearate blend (for example, ARLACEL®165), mineral oil, diisopropyl adipate and petrolatum, then adding spironolactone and a preservative such as propyl-p-hydroxybenzoate and homogenizing the ingredients. The homogeneous blend is then emulsified with the following aqueous phase ingredients which have been mixed together and heated; sodium citrate, citric acid, a preservative, such as methyl-p-hydroxybenzoate, and water. Following emulsification, the mixture is cooled, then blended to produce the final spironolactone product.

The stability of a spironolactone 5% cream having the formulation shown in Example 2 was studied in glass jars. Stability data generated on samples stored at accelerated conditions (50°C, 40°C, cyclic temperatures of -10°C to 40°C and intense light) and at room temperature indicated little change for both percent spironolactone and percent canrenone, which is a decomposition product of spironolactone, after 13 weeks. Samples stored at room temperature showed little change after 13, 21, 39 and 58 weeks.

Two trials were initiated to evaluate the stability of a spironolactone 5% cream topical preparation having the formulation shown in Example 2 packaged in laminate tubes with polypropylene caps. Conditions of storage included 40°C and 30°C, with the, inner seal of the tube punctured and unpunctured. Assays for spironolactone and canrenone, as well as pH and physical characteristics (color, odor, and appearance) were evaluated. Samples packaged in laminate tubes with polypropylene caps and stored with the inner seal punctured and unpunctured at 40°C and 30°C showed no change through the 13 week test interval. Physical characteristics remained unchanged.

A sample prepared according to U.S. 4,543,351 (Messina) Formulation B (column 3, Example 2) which was stored at 50°C for one week showed some separation and had a gritty feel when applied to the skin.

The following formulation examples illustrate the compositions of the present invention and are not to be construed as limiting the invention either in spirit or in scope. It will be obvious to those skilled in the art that many modifications thereof may be made without departing from the purpose and intent of this disclosure. In these examples, temperatures are given in degrees Celcius (°C) and quantities of materials in grams and milliliters unless otherwise noted. All percentages are by weight.

## EXAMPLE I

A 5% w/w topical spironolactone cream is prepared from the following ingredients.

| INGREDIENTS | QUANTITY, g. |
|---|---|
| **OIL PHASE** | |
| Spironolactone, USP, micronized | 150.0 |
| Cetearyl Alcohol and Sodium Lauryl Sulfate Mixture[1] | 270.0 |
| Glycerol Monostearate and Polyoxyethylene Stearate Mixture[2] | 150.0 |
| Light Mineral Oil, NF | 180.0 |
| Diisopropyl Adipate | 135.0 |
| White Petrolatum, USP | 315.0 |
| Propyl-p-Hydroxybenzoate, NF (Propyl Paraben) | 1.5 |
| **AQUEOUS PHASE** | |
| Methyl-p-Hydroxybenzoate, NF (Methyl Paraben) | 4.5 |
| Sodium Citrate Dihydrate, USP | 19.5 |
| Citric Acid Monohydrate, USP | 15.0 |
| Purified Water, USP | 1760.0 |

(1) Lanette SX, Henkel Corp., Ambler, PA
(2) Arlacel®165, ICI Americas, Inc., New Castle, Delaware

## PROCEDURE

1. In a suitable vessel charge the white petrolatum; light mineral oil, diisopropyl adipate, cetearyl alcohol and sodium lauryl sulfate mixture, and glycerol monostearate and polyoxyethylene stearate mixture. Melt and heat to about 80 to 82°C. This is the oil phase.
2. In another suitable vessel, charge the methyl paraben, sodium citrate dihydrate, citric acid monohydrate, and purified water and heat to about 80 to 82°C with agitation. This is the aqueous phase.
3. After all materials are melted and the temperature of the oil phase reaches about 80 to 82°C, add the propyl paraben and spironolactone to the oil phase.
4. Homogenize the oil phase and then combine the oil phase with the aqueous phase under vacuum using homogenizer and agitator speeds suitable to provide the desired emulsification.
5. Cool with gradually reduced agitation until the product temperature reaches about 25°C; then transfer the product to an appropriate container.

EXAMPLE 2

Using the procedure of Example 1, formulations containing varying amounts of the spironolactone active ingredient can be prepared. The following shows the amounts in weight percent of the various ingredients used to make formulations containing 1%, 3% or 5% of spironolactone.

| INGREDIENTS | % (W/W) | | |
|---|---|---|---|
| | 5% CREAM | 3% CREAM | 1% CREAM |
| **OIL PHASE** | | | |
| Spironolactone, USP micronized | 5.0 | 3.0 | 1.0 |
| Cetearyl Alcohol and Sodium Lauryl Sulfate Mixture[1] | 9.0 | 9.0 | 9.0 |
| Glycerol Monostearate and Polyoxyethylene Stearate Mixture[2] | 5.0 | 5.0 | 5.0 |
| Light Mineral Oil, NF | 6.0 | 6.0 | 6.0 |
| Diisopropyl Adipate | 4.5 | 4.5 | 4.5 |
| White Petrolatum, USP | 10.5 | 10.5 | 10.5 |
| Propyl-p-Hydroxybenzoate, NF (Propyl Paraben) | 0.05 | 0.05 | 0.05 |
| **AQUEOUS PHASE** | | | |
| Methyl-p-Hydroxybenzoate, NF (Methyl Paraben) | 0.15 | 0.15 | 0.15 |
| Sodium Citrate Dihydrate, USP | 0.65 | 0.65 | 0.65 |
| Citric Acid Monohydrate, USP | 0.5 | 0.5 | 0.5 |
| Purified Water, USP | 58.65 | 60.65 | 62.65 |

(1) Lanette SX
(2) Arlacel® 165

**Claims**

1. A topical composition comprising:
    (a) from about 1.0 to about 12.0 weight percent of spironolactone;
    (b) from about 2.5 to about 18.5 weight percent of emulsifying agents;
    (c) from about 0.5 to about 6.0 weight percent of solvent;
    (d) buffering agents sufficient to maintain a pH of from about 4.0 to about 6.0; and
    (e) from about 50 to about 80 weight percent water.
2. A composition according to Claim 1 comprising:
    (a) from about 1.0 to about 12.0 weight percent of spironolactone;
    (b) from about 2.5 to about 18.5 weight percent of a mixed cetearyl alcohol and sodium lauryl sulfate and a mixed glycerol monostearate and polyoxyethylene stearate;
    (c) from about 0.5 to about 6.0 weight percent of solvent selected from the group consisting of diisopropyl adipate and $C_8$-$C_{10}$ ethoxylated glyceride;
    (d) from about 0.2 to about 2.3 weight percent of buffering agents; and
    (e) from about 50 to about 80 weight percent of water.
3. A composition according to Claim 1 comprising:
    (a) from about 1.0 to about 12.0 weight percent of spironolactone;
    (b) from about 1.5 to about 12.0 weight percent of a mixture of cetearyl alcohol and sodium lauryl sulfate;
    (c) from about 1.0 to about 6.5 weight percent of a mixture of glycerol monostearate and polyoxyethylene stearate;
    (d) from about 0.5 to about 6.0 weight percent of diisopropyl adipate;
    (e) from about 0.13 to about 1.3 weight percent of a weak acid salt;

(f) from about 0.1 to about 1.0 weight percent of a weak acid; and

(g) from about 50 to about 80 weight percent of water.

4. A composition according to Claim 1 further containing a preservative selected from the group consisting of methyl-p-hydroxybenzoate, ethyl-p-hydroxybenzoate, propyl-p-hydroxybenzoate, butyl-p-hydroxybenzoate, benzoic acid, and imidazolidinyl urea.

5. A composition according to Claim 4 further containing:

(a) from about 1.0 to about 12.0 weight percent of a noncomedogenic liquid carrier; and

(b) from about 1.0 to about 15.0 weight percent of an ointment base.

6. A composition according to Claim 5 wherein the noncomedogenic liquid carrier is mineral oil and the ointment base is petrolatum.

7. A topical composition comprising:

(a) from about 1.0 to about 12.0 weight percent of spironolactone;

(b) from about 1.5 to about 12.0 weight percent of a mixture of cetearyl alcohol and sodium lauryl sulfate;

(c) from about 1.0 to about 6.5 weight percent of a mixture of glycerol monostearate and polyoxyethylene stearate;

(d) from about 1.0 to about 12.0 weight percent of mineral oil;

(e) from about 0.5 to about 6.0 weight percent of solvent selected from the group consisting of diisopropyl adipate and $C_8$-$C_{10}$ ethoxylated glyceride;

(f) from about 1.0 to about 15.0 weight percent of petrolatum;

(g) from about 0.02 to about 0.3 weight percent of propyl-p-hydroxybenzoate;

(h) from about 0.02 to about 0.3 weight percent of methyl-p-hydroxybenzoate;

(i) from about 0.13 to about 1.3 weight percent of a weak acid salt;

(j) from about 0.1 to about 1.0 weight percent of a weak acid; and

(k) from about 50 to about 80 weight percent of water.

8. A topical composition according to Claim 7 comprising:

(a) from about 1.0 to about 12.0 weight percent of spironolactone;

(b) from about 7.2 to about 10.8 weight percent of a mixture of cetearyl alcohol and sodium lauryl sulfate;

(c) from about 4.0 to about 6.0 weight percent of a mixture of glycerol monostearate and polyoxyethylene stearate;

(d) from about 4.8 to about 7.2 weight percent of light mineral oil;

(e) from about 3.6 to about 5.4 weight percent of diisopropyl adipate;

(f) from about 8.4 to about 12.6 weight percent of white petrolatum;

(g) from about 0.04 to about 0.06 weight percent of propyl-p-hydroxybenzoate;

(h) from about 0.12 to about 0.18 weight percent of methyl-p-hydroxybenzoate;

(i) from about 0.53 to about 0.78 weight percent of sodium citrate dihydrate;

(j) from about 0.4 to about 0.6 weight percent of citric acid monohydrate; and

(k) from about 55 to about 70 weight percent of water.

9. A topical composition according to Claim 8 comprising:

(a) from about 1.0 to about 5.0 weight percent of spironolactone;

(b) about 9.0 weight percent of a mixture of cetearyl alcohol and sodium lauryl sulfate;

(c) about 5.0 weight percent of a mixture of glycerol monostearate and polyoxyethylene stearate;

(d) about 6.0 weight percent of light mineral oil;

(e) about 4.5 weight percent of diisopropyl adipate;

(f) about 10.5 weight percent of white petrolatum;

(g) about 0.05 weight percent of propyl-p-hydroxybenzoate;

(h) about 0.15 weight percent of methyl-p-hydroxybenzoate;

(i) about 0.65 weight percent of sodium citrate dihydrate;

(j) about 0.5 weight percent of citric acid monohydrate; and

(k) from about 62.65 to about 58.65 weight percent of water.

10. A topical composition according to Claim 8 comprising:

(a) about 5.0 weight percent of spironolactone;

(b) about 9.0 weight percent of a mixture of cetearyl alcohol and sodium lauryl sulfate;

(c) about 5.0 weight percent of a mixture of glycerol monostearate and polyoxyethylene stearate;

(d) about 6.0 weight percent of light mineral oil;

(e) about 4.5 weight percent of diisopropyl adipate;

(f) about 10.5 weight percent of white petrolatum;

(g) about 0.05 weight percent of propyl-p-hydroxybenzoate;

(h) about 0.15 weight percent of methyl-p-hydroxybenzoate;

(i) about 0.65 weight percent of sodium citrate dihydrate;

(j) about 0.5 weight percent of citric acid monohydrate; and

(k) about 58.65 weight percent of water.

11. A topical composition according to Claim 8 comprising:

(a) about 3.0 weight percent of spironolactone;

(b) about 9.0 weight percent of a mixture of cetearyl alcohol and sodium lauryl sulfate;

(c) about 5.0 weight percent of a mixture of glycerol monostearate and polyoxyethylene stearate;

(d) about 6.0 weight percent of light mineral oil;

(e) about 4.5 weight percent of diisopropyl adipate;

(f) about 10.5 weight percent of white petrolatum;

(g) about 0.05 weight percent of propyl-p-hydroxybenzoate;

(h) about 0.15 weight percent of methyl-p-hydroxybenzoate;

(i) about 0.65 weight percent of sodium citrate dihydrate;

(j) about 0.5 weight percent of citric acid monohydrate; and

(k) about 60.65 weight percent of water.

12. A topical composition according to Claim 8 comprising:

(a) about 1.0 weight percent of spironolactone;

(b) about 9.0 weight percent of a mixture of cetearyl alcohol and sodium lauryl sulfate;

(c) about 5.0 weight percent of a mixture of glycerol monostearate and polyoxyethylene stearate;

(d) about 6.0 weight percent of light mineral oil;

(e) about 4.5 weight percent of diisopropyl adipate;

(f) about 10.5 weight percent of white petrolatum;

(g) about 0.05 weight percent of propyl-p-hydroxybenzoate;

(h) about 0.15 weight percent of methyl-p-hydroxybenzoate;

(i) about 0.65 weight percent of sodium citrate dihydrate;

(j) about 0.5 weight percent of citric acid monohydrate; and

(k) about 62.65 weight percent of water.

13. A topical composition according to Claim 8 wherein the mixture of cetearyl alcohol and sodium lauryl sulfate is about 90% cetearyl alcohol and about 10% sodium lauryl sulfate, and the mixture of glycerol monostearate and polyoxyethylene stearate is about 50% glycerol monostearate and about 50% polyoxyethylene stearate.

14. A topical composition according to Claim 9 wherein the mixture of cetearyl alcohol and sodium lauryl sulfate is about 90% cetearyl alcohol and about 10% sodium lauryl sulfate, and the mixture of glycerol monostearate and polyoxyethylene stearate is about 50% glycerol monostearate and 50% polyoxyethylene stearate.

15. A topical composition according to Claim 10 wherein the mixture of cetearyl alcohol and sodium lauryl sulfate is about 90% cetearyl alcohol and about 10% sodium lauryl sulfate, and the mixture of glycerol monostearate and polyoxyethylene stearate is about 50% glycerol monostearate and 50% polyoxyethylene stearate.

16. A topical composition according to Claim 11 wherein the mixture of cetearyl alcohol and sodium lauryl sulfate is about 90% cetearyl alcohol and about 10% sodium lauryl sulfate, and the mixture of glycerol monostearate and polyoxyethylene stearate is about 50% glycerol monostearate and 50% polyoxyethylene stearate.

17. A topical composition according to Claim 12 wherein the mixture of cetearyl alcohol and sodium lauryl sulfate is about 90% cetearyl alcohol and about 10% sodium lauryl sulfate, and the mixture of glycerol monostearate and polyoxyethylene stearate is about 50% glycerol monostearate and 50% polyoxyethylene stearate.

18. A process for preparing a topical spironolactone composition comprising:

(a) forming an oil phase by heating a mixture of a mixed cetearyl alcohol and sodium lauryl sulfate, a mixed glycerol monostearate and polyoxyethylene stearate, mineral oil, diisopropyl adipate and petrolatum until melted;

(b) forming an aqueous phase by mixing together and heating an acid component, an acid salt component, a preservative component and water;

(c) adding spironolactone and a preservative component to the oil phase and homogenizing the ingredients;

(d) emulsifying the homogeneous oil phase with the aqueous phase under vacuum using homogenizer and agitator speeds suitable to provide the desired emulsification; and

(e) cooling and blending the emulsified mixture with agitation to produce the product.

19. A process according to Claim 18 for preparing a topical spironolactone cream which comprises:

(a) forming an oil phase by heating until melted a mixture of white petrolatum, mineral oil, diisopropyl adipate, a cetearyl alcohol and sodium lauryl sulfate mixture, and a glycerol monostearate and polyoxyethylene stearate mixture;

(b) forming an aqueous phase by heating a mixture of methyl paraben, sodium citrate, citric acid and water;

(c) adding propyl paraben and spironolactone to the oil phase and homogenizing the oil phase;

(d) emulsifying the homogeneous oil phase with the aqueous phase by adding the oil phase to the aqueous phase under a vacuum with simultaneous homogenization and agitation at speeds suitable for producing the desired emulsification while maintaining a temperature sufficient to keep the oil phase ingredients in a melted form;

(e) cooling the emulsion gradually to about 25°C while blending the mixture by gradually reducing the agitation to produce the product.

20. Use of 1 to 12 weight percent of spironolactone, 0.5 to 6 weight percent of a solvent selected from the group of diisopropyl adipate and $C_8$-$C_{10}$ ethoxylated glyceride as well as suitable carriers such as emulsifying and buffering agents and water for preparing a medicament for treating a skin site having excess androgenic activity.

21. Use according to Claim 20 wherein the excess androgenic activity is acne.

22. Use according to Claim 20 wherein the excess androgenic activity is hirsutism.

23. Use according to Claim 20 wherein the excess androgenic activity is seborrhea.

24. Use according to Claim 20 wherein the excess androgenic activity is alopecia.

Claims for the following Contracting States: ES, GR

1. A process for preparing a topical spironolactone composition comprising:

(a) forming an oil phase by heating a mixture of a mixed cetearyl alcohol and sodium lauryl sulfate, a mixed glycerol monostearate and polyoxyethylene stearate, mineral oil, diisopropyl adipate and petrolatum until melted;

(b) forming an aqueous phase by mixing together and heating an acid component, an acid salt component, a preservative component and water;

(c) adding spironolactone and a preservative component to the oil phase and homogenizing the ingredients;

(d) emulsifying the homogeneous oil phase with the aqueous phase under vacuum using homogenizer and agitator speeds suitable to provide the desired emulsification; and

(e) cooling and blending the emulsified mixture with agitation to produce the product.

2. A process according to Claim 1 for preparing a topical spironolactone cream which comprises:

(a) forming an oil phase by heating until melted a mixture of white petrolatum, mineral oil, diisopropyl adipate, a cetearyl alcohol and sodium lauryl sulfate mixture, and a glycerol monostearate and polyoxyethylene stearate mixture;

(b) forming an aqueous phase by heating a mixture of methyl paraben, sodium citrate, citric acid and water;

(c) adding propyl paraben and spironolactone to the oil phase and homogenizing the oil phase;

(d) emulsifying the homogeneous oil phase with the aqueous phase by adding the oil phase to the aqueous phase under a vacuum with simultaneous homogenization and agitation at speeds suitable for producing the desired emulsification while maintaining a temperature sufficient to keep the oil phase ingredients in a melted form;

(e) cooling the emulsion gradually to about 25°C while blending the mixture by gradually reducing the agitation to produce the product.

3. Process according to Claim 1 wherein the composition prepared comprises:

(a) from about 1.0 to about 12.0 weight percent of spironolactone;

(b) from about 2.5 to about 18.5 weight percent of emulsifying agents;

(c) from about 0.5 to about 6.0 weight percent of solvent;

(d) buffering agents sufficient to maintain a pH of from about 4.0 to about 6.0; and

(e) from about 50 to about 80 weight percent water.

4. Process according to Claim 1 wherein the composition prepared comprises:

(a) from about 1.0 to about 12.0 weight percent of spironolactone;

(b) from about 2.5 to about 18.5 weight percent of a mixed cetearyl alcohol and sodium lauryl sulfate and a mixed glycerol monostearate and polyoxyethylene stearate;

(c) from about 0.5 to about 6.0 weight percent of solvent selected from the group consisting of diisopropyl adipate and $C_8$-$C_{10}$ ethoxylated glyceride;

(d) from about 0.2 to about 2.3 weight percent of buffering agents; and

(e) from about 50 to about 80 weight percent of water.

5. Process according to Claim 1 wherein the composition prepared comprises:

(a) from about 1.0 to about 12.0 weight percent of spironolactone;

(b) from about 1.5 to about 12.0 weight percent of a mixture of cetearyl alcohol and sodium lauryl sulfate;

(c) from about 1.0 to about 6.5 weight percent of a mixture of glycerol monostearate and polyoxyethylene stearate;

(d) from about 0.5 to about 6.0 weight percent of diisopropyl adipate;

(e) from about 0.13 to about 1.3 weight percent of a weak acid salt;

(f) from about 0.1 to about 1.0 weight percent of a weak acid; and

(g) from about 50 to about 80 weight percent of water.

6. Process according to Claim 1 wherein the composition further comprises a preservative selected from the group consisting of methyl-p-hydroxybenzoate, ethyl-p-hydroxybenzoate, propyl-p-hydroxybenzoate, butyl-p-hydroxybenzoate, benzoic acid, and imidazolidinyl urea.

7. Process according to Claim 6 wherein the composition further comprises:

(a) from about 1.0 to about 12.0 weight percent of a noncomedogenic liquid carrier; and

(b) from about 1.0 to about 15.0 weight percent of an ointment base.

8. Process according to Claim 7 wherein the noncomedogenic liquid carrier is mineral oil and the ointment base is petrolatum.

9. Process according to Claim 1 wherein the composition prepared comprises:

(a) from about 1.0 to about 12.0 weight percent of spironolactone;

(b) from about 1.5 to about 12.0 weight percent of a mixture of cetearyl alcohol and sodium lauryl sulfate;

(c) from about 1.0 to about 6.5 weight percent of a mixture of glycerol monostearate and polyoxyethylene stearate;

(d) from about 1.0 to about 12.0 weight percent of mineral oil;

(e) from about 0.5 to about 6.0 weight percent of solvent selected from the group consisting of diisopropyl adipate and $C_8$-$C_{10}$ ethoxylated glyceride;

(f) from about 1.0 to about 15.0 weight percent of petrolatum;

(g) from about 0.02 to about 0.3 weight percent of propyl-p-hydroxybenzoate;

(h) from about 0.02 to about 0.3 weight percent of methyl-p-hydroxybenzoate;

(i) from about 0.13 to about 1.3 weight percent of a weak acid salt;

(j) from about 0.1 to about 1.0 weight percent of a weak acid; and

(k) from about 50 to about 80 weight percent of water.

10. Process according to Claim 9 wherein the composition prepared comprises:

(a) from about 1.0 to about 12.0 weight percent of spironolactone;

(b) from about 7.2 to about 10.8 weight percent of a mixture of cetearyl alcohol and sodium lauryl sulfate;

(c) from about 4.0 to about 6.0 weight percent of a mixture of glycerol monostearate and polyoxyethylene stearate;

(d) from about 4.8 to about 7.2 weight percent of light mineral oil;

(e) from about 3.6 to about 5.4 weight percent of diisopropyl adipate;

(f) from about 8.4 to about 12.6 weight percent of white petrolatum;

(g) from about 0.04 to about 0.06 weight percent of propyl-p-hydroxybenzoate;

(h) from about 0.12 to about 0.18 weight percent of methyl-p-hydroxybenzoate;

(i) from about 0.53 to about 0.78 weight percent of sodium citrate dihydrate;

(j) from about 0.4 to about 0.6 weight percent of citric acid monohydrate; and

(k) from about 55 to about 70 weight percent of water.

11. Process according to Claim 10 wherein the composition prepared comprises:

(a) from about 1.0 to about 5.0 weight percent of spironolactone;

(b) about 9.0 weight percent of a mixture of cetearyl alcohol and sodium lauryl sulfate;

(c) about 5.0 weight percent of a mixture of glycerol monostearate and polyoxyethylene stearate;

(d) about 6.0 weight percent of light mineral oil;

(e) about 4.5 weight percent of diisopropyl adipate;

(f) about 10.5 weight percent of white petrolatum;

(g) about 0.05 weight percent of propyl-p-hydroxybenzoate;

(h) about 0.15 weight percent of methyl-p-hydroxybenzoate;

(i) about 0.65 weight percent of sodium citrate dihydrate;

(j) about 0.5 weight percent of citric acid monohydrate; and

(k) from about 62.65 to about 58.65 weight percent of water.

12. Process according to Claim 10 wherein the compositon prepared comprises:

(a) about 5.0 weight percent of spironolactone;

(b) about 9.0 weight percent of a mixture of cetearyl alcohol and sodium lauryl sulfate;

(c) about 5.0 weight percent of a mixture of glycerol monostearate and polyoxyethylene stearate;

(d) about 6.0 weight percent of light mineral oil;

(e) about 4.5 weight percent of diisopropyl adipate;

(f) about 10.5 weight percent of white petrolatum;

(g) about 0.05 weight percent of propyl-p-hydroxybenzoate;

(h) about 0.15 weight percent of methyl-p-hydroxybenzoate;

(i) about 0.65 weight percent of sodium citrate dihydrate;

(j) about 0.5 weight percent of citric acid monohydrate; and

(k) about 58.65 weight percent of water.

13. Process according to Claim 10 wherein the composition prepared comprises:

(a) about 3.0 weight percent of spironolactone;

(b) about 9.0 weight percent of a mixture of cetearyl alcohol and sodium lauryl sulfate;

(c) about 5.0 weight percent of a mixture of glycerol monostearate and polyoxyethylene stearate;

(d) about 6.0 weight percent of light mineral oil;

(e) about 4.5 weight percent of diisopropyl adipate;

(f) about 10.5 weight percent of white petrolatum;

(g) about 0.05 weight percent of propyl-p-hydroxybenzoate;

(h) about 0.15 weight percent of methyl-p-hydroxybenzoate;

(i) about 0.65 weight percent of sodium citrate dihydrate;

(j) about 0.5 weight percent of citric acid monohydrate; and

(k) about 60.65 weight percent of water.

14. Process according to Claim 10 wherein the compositions prepared comprises:

(a) about 1.0 weight percent of spironolactone;

(b) about 9.0 weight percent of a mixture of cetearyl alcohol and sodium lauryl sulfate;

(c) about 5.0 weight percent of a mixture of glycerol monostearate and polyoxyethylene stearate;

(d) about 6.0 weight percent of light mineral oil;

(e) about 4.5 weight percent of diisopropyl adipate;

(f) about 10.5 weight percent of white petrolatum;

(g) about 0.05 weight percent of propyl-p-hydroxybenzoate;

(h) about 0.15 weight percent of methyl-p-hydroxybenzoate;

(i) about 0.65 weight percent of sodium citrate dihydrate;

(j) about 0.5 weight percent of citric acid monohydrate; and

(k) about 62.65 weight percent of water.

15. Process according to Claim 10 wherein the mixture of cetearyl alcohol and sodium lauryl sulfate is about 90% cetearyl alcohol and about 10% sodium lauryl sulfate, and the mixture of glycerol monostearate and polyoxyethylene stearate is about 50% glycerol monostearate and about 50% polyoxyethylene stearate.

16. Process according to Claim 11 wherein the mixture of cetearyl alcohol and sodium lauryl sulfate is about 90% cetearyl alcohol and about 10% sodium lauryl sulfate, and the mixture of glycerol monostearate and polyoxyethylene stearate is about 50% glycerol monostearate and 50% polyoxyethylene stearate.

17. Process according to Claim 12 wherein the mixture of cetearyl alcohol and sodium lauryl sulfate is about 90% cetearyl alcohol and about 10% sodium lauryl sulfate, and the mixture of glycerol monostearate and polyoxyethylene stearate is about 50% glycerol monostearate and 50% polyoxyethylene stearate.

18. Process according to Claim 13 wherein the mixture of cetearyl alcohol and sodium lauryl sulfate is about 90% cetearyl alcohol and about 10% sodium lauryl sulfate, and the mixture of glycerol monostearate and polyoxyethylene stearate is about 50% glycerol monostearate and 50% polyoxyethylene stearate.

19. Process according to Claim 14 wherein the mixture of cetearyl alcohol and sodium lauryl sulfate is about 90% cetearyl alcohol and about 10% sodium lauryl sulfate, and the mixture of glycerol monostearate and polyoxyethylene stearate is about 50% glycerol monostearate and 50% polyoxyethylene stearate.

20. Use of 1 to 12 weight percent of spironolactone, 0.5 to 6 weight percent of a solvent selected from the group of diisopropyl adipate and $C_8$-$C_{10}$ ethoxylated glyceride as well as suitable carriers such as emulsifying and buffering agents and water for preparing a medicament for treating a skin site having excess androgenic activity.

12

21. Use according to Claim 20 wherein the excess androgenic activity is acne.
22. Use according to Claim 20 wherein the excess androgenic activity is hirsutism.
23. Use according to Claim 20 wherein the excess androgenic activity is seborrhea.
24. Use according to Claim 20 wherein the excess androgenic activity is alopecia.

European
Patent Office

EUROPEAN SEARCH
REPORT

Application Number

EP 90 11 4076

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,A,D | EP-A-0 124 147  (SCHIAPPARELLI FARMACEUTICI) <br> * claims 1-5 * * claims& US-A-4543351 * <br> − − − | 1,4,2-3, 5-24 | A 61 K 31/585 <br> A 61 K 7/48 |
| A,D | FR-A-2 588 755  (ARON-BRUNETIERE) <br> * the whole document * <br> − − − | 1-24 | |
| A,D | US-A-4 347 245  (SHAPIRO G.) <br> * the whole document * <br> − − − | 1-24 | |
| A,D | US-A-3 711 602  (HERSCHLER R.J.) <br> * column 10 * example 16 * <br> − − − | 1-24 | |
| A | US-A-4 837 211  (OLSEN J.L.) <br> * the whole document * <br> − − − | 1-24 | |
| A | US-A-4 684 635  (ORENTREICH N.) <br> * the whole document * <br> − − − | 1-24 | |
| A,D | EP-A-0 028 525  (ORION-YHTYMÄ OY) <br> * the whole document * <br> − − − − − | 1-24 | |

| TECHNICAL FIELDS SEARCHED (Int. Cl.5) |
|---|
| A 61 K |

The present search report has been drawn up for all claims

| Place of search | Date of completion of search | Examiner |
|---|---|---|
| The Hague | 29 October 90 | AVEDIKIAN P.F. |